# FASCICULE DE BREVET EUROPEEN

(11) **EP 0 279 745 B1**
(45) Date de publication et mention de la délivrance du brevet: **02.11.1994**
(21) Numéro de dépôt: 88400349.2
(22) Date de dépôt: 17.02.1988
(51) Int. Cl.: A61B 17/36, A61B 17/39, H05H 1/34

(54) **Bistouri à plasma**
Plasma-Operationsmesser
Plasma scalpel

(30) Priorité: 19.02.1987 FR 8702197
(43) Date de publication de la demande: 24.08.1988
(73) Titulaire: UNIVERSITE RENE DESCARTES (PARIS V), F-75270 Paris Cédex 06 (FR)
(72) Inventeur: Bouchier, Guy, F-77440 Lizy sur Ourcq (FR); Lhuisset, François, F-91800 Brunoy (FR)
(74) Mandataire: Pinguet, André

(56) Documents cités:
- FR-A- 1 207 809
- FR-A- 1 565 713
- FR-E- 57 862
- GB-A- 1 135 389
- US-A- 3 158 727
- US-A- 3 991 764
- US-A- 4 009 413
- US-A- 4 057 064

## Description

La présente invention concerne un procédé de découpage et/ou de traitement de matières dures ou molles, notamment des tissus vivants, par production d'un courant de gaz ionise -ou plasma- au voisinage, à la surface ou dans lesdites matières ou ledit tissu. L'invention concerne également un dispositif, dit bistouri ou microchalumeau à plasma, pour mettre en oeuvre ce procédé.

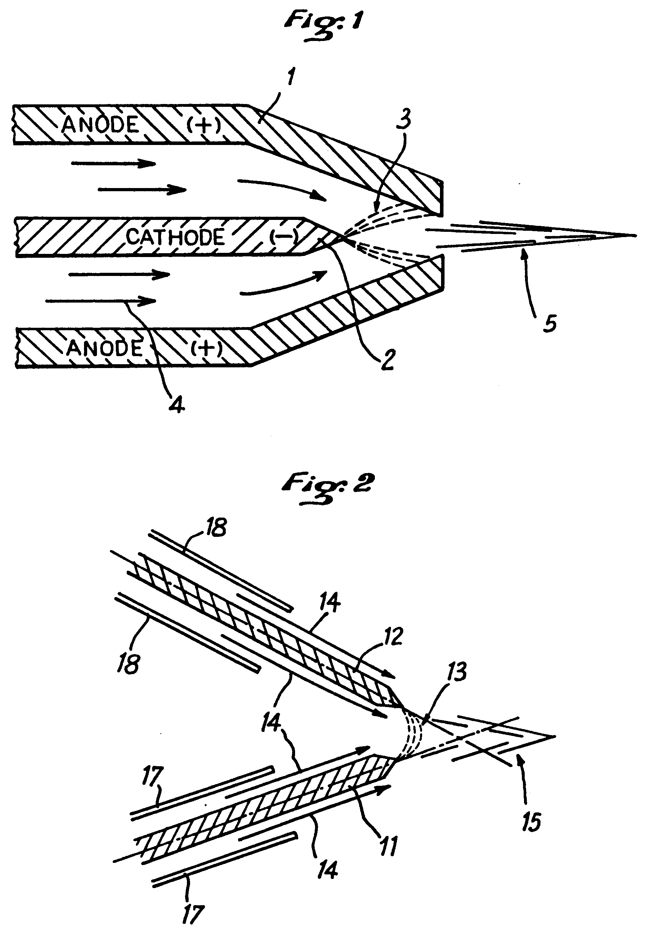

Le principe de découper ou travailler des matières par production d'un plasma -ou gaz fortement ionisé- n'est pas nouveau en lui-même. Du plasma peut être créé en faisant passer un gaz à travers un arc électrique. L'énergie électrique dans l'arc est convertie en énergie thermique dans le gaz, et le gaz, après être passé à travers l'arc électrique, se présente sous la forme d'un plasma partiellement ionisé à haute température. Dans le domaine de la médecine, des prototypes expérimentaux de bistouri à plasma ont été réalisés à partir de deux électrodes coaxiales, une anode extérieure formant tuyère et une cathode intérieure se terminant en pointe légèrement en retrait par rapport à l'extrémité de l'anode extérieure. Un courant de gaz parcourant l'espace intérieur ainsi créé entre l'électrode extérieure et l'électrode intérieure passe à travers l'arc électrique produit aux extrémités des électrodes et ressort sous la forme d'un plasma à haute température. Cette disposition est illustrée par le schéma de la figure 1, où on reconnaît l'anode extérieure en forme de tuyère 1 et la cathode intérieure en forme d'aiguille 2. On a également symbolisé quelques arcs électriques par des traits interrompus 3, et le courant de gaz par des flèches 4, ainsi que la sortie de plasma 5. Un tel prototype de bistouri à plasma a été développé par Link et al. à l'Université de Purdue en 1970. Le jet de plasma à haute température issu de la tuyère de ce prototype de bistouri à plasma est engendré en faisant passer de l'argon à travers un arc électrique de courant continu. L'arc électrique est amorcé par une mise en contact des deux électrodes. Les températures moyennes du plasma, à la sortie de la tuyère, sont approximativement de 3000°C.

Ce dispositif de l'art antérieur présente cependant quelques inconvénients. Pendant la production du plasma, l'anode atteint des températures très élevées, et nécessite un refroidissement important, tandis que la cathode n'a pas besoin d'un refroidissement aussi important. L'usage d'un système propulsant l'arc par jet gazeux nettement en dehors des électrodes étale le phénomène dans l'espace ce qui empêche des températures très élevées ; le refroidissement dû à l'effet de détente gazeuse cumulé avec le refroidissement naturel très rapide du plasma, puisqu'il n'est plus excité, semble la cause de ce phénomène. L'usage de gaz rare est de moins en moins répandu car coûteux et avec un mauvais coefficient de transfert thermique.

Le document US-A-4.057.064 décrit un bistouri à plasma ayant deux électrodes coaxiales soumises à des différences de potentiel alternatives haute fréquence. Un flux de gaz s'écoule entre les deux électrodes.

Le document FR-A-1.207.809 décrit un chalumeau à plasma, comportant au moins deux électrodes espacées soumises à des différences de potentiel électrique alternatives telles que des arcs électriques se produisent entre elles. Les extrémités desdites électrodes sont allongées et forment entre elles un angle aigu, des courants de fluide arrivant le long desdites électrodes.

Le document FR-A-1.565.713 divulgue un chalumeau à plasma ayant une bobine haute fréquence qui permet d'élever la tension électrique délivrée à une électrode.

Le document US-A-4.009.413 décrit un chalumeau à plasma dont les électrodes forment entre elles un angle aigu, un courant de gaz arrivant le long de chaque électrode et depuis l'intérieur dudit angle.

La présente invention a pour but d'améliorer les bistouris à plasma existants, tels que celui divulgué par le document US-A-4,057,064, dans les domaines suivants :
- sécurité d'utilisation, et notamment suppression des risques de brûlures dus au câble d'alimentation,
- limitation des pertes de puissance dans le câble,
- symétrie d'usure et d'échauffement des électrodes,
- précision de la localisation, et
- polyvalence.

Selon l'invention, ces buts sont atteints par un bistouri à plasma comportant une poignée, un câble d'alimentation électrique et au moins deux électrodes espacées soumises à des différences de potentiel électrique telles que des arcs électriques se produisent entre elles et un moyen pour produire au moins un courant de fluide traversant une région de l'espace où lesdits arcs électriques ont lieu, et lesdites électrodes étant adaptées à être mises sensiblement en contact avec une zone à traiter, lesdites différences de potentiel étant alternatives, de moyenne ou haute fréquence, caractérisé en ce que les extrémités desdites électrodes sont filiformes ou pointues et forment entre elles un angle aigu, et un transformateur élévateur de tension est incorporé au bistouri pour délivrer lesdites différences de potentiel alternatives, lesdits courants de fluide arrivant depuis l'intérieur dudit angle, ou le long d'au moins une desdites électrodes. Le bistouri en question est un outil ou un instrument maniable, en principe avec une seule main. Le fait que les différences de potentiel soient de fréquence moyenne ou haute les rend sans risque d'électrocution pour un usage médical ou dentaire.

Le dispositif peut ne comporter que deux électrodes avec une alimentation en courant monophasé amenant à la réalisation d'un plasma alternatif. Il peut également comporter plus de deux électrodes et l'alimentation être polyphasée ; dans le cas de trois ou six électrodes par exemple, l'alimentation des électrodes peut être triphasée.

Avantageusement, les extrémités des électrodes -ou les électrodes elles-mêmes-ont une forme allongée, ou une faible section, de façon à favoriser un effet de pointe tendant à orienter les extrémités de l'arc électrique dans l'alignement des extrémités des électrodes. Dans ce but, les électrodes peuvent se présenter sous la forme de fils.

Le courant de fluide a plusieurs fonctions : il dirige le jet de plasma et refroidit les électrodes et/ou le matériau pendant le fonctionnement du dispositif. Il peut également participer à la formation du plasma.

Le but d'un tel dispositif est donc de produire du plasma dans un petit volume par décharge électrique dirigée par effet de pointe et/ou par déplacement de fluide pour une application sur la surface desdites matières ou desdits tissus, pour pouvoir ainsi les creuser, les découper ou, d'une façon générale, les traiter par chauffage rapide à très haute température. Lorsque l'on découpe un tissu vivant avec un tel dispositif, les températures de travail sont suffisamment élevées pour que l'opération soit très rapide, c'est-à-dire par passes de quelques fractions de secondes, de telle sorte que le tissu n'a pas le temps de s'échauffer en profondeur. Il en résulte, dans le cas des tissus vivants, que la douleur est moindre, car l'effet thermique reste très superficiel, et que la cicatrisation ultérieure du tissu est rapide, comme le montrent des essais réalisés en laboratoire sur des rats ou des lapins. En d'autres termes, le gradient thermique étant très élevé et les temps d'application pouvant être extrêmement courts, la zone thermique affectée (zone coagulée pour les tissus vivants) sera très réduite, ce qui explique les bons résultats.

Les électrodes sortant suffisamment des porte-électrodes se retrouvent à leur extrémité à une courte distance l'une de l'autre et en quasi contact, voire en contact avec la zone à traiter, d'où un exceptionnel gradient de température très favorable pour réduire la zone thermique affectée. On a la possibilité de travail en appui sur la paroi à traiter ce qui est nécessaire à la précision de l'acte lors du guidage manuel. Ces détails sont très importants, par exemple en chirurgie pour la fusion de tissus durs ou la volatilisation de tissus mous ; dans ce cas, cette configuration impose l'usage d'un courant nécessairement alternatif à fréquence suffisamment élevée pour son innocuité vis-à-vis d'êtres vivants et/ou un système élévateur de tension assez petit, sa taille pour une puissance donnée étant liée à une fréquence élevée.

La présente invention a aussi pour objet un procédé d'utilisation du bistouri susmentionné, caractérisé en ce qu'il comprend les étapes suivantes :
- ouvrir l'alimentation en air pour mettre la chambre sous pression d'air, et provoquer ainsi un courant d'air le long ou autour des électrodes;
- établir un courant haute fréquence pour amorcer l'arc électrique à l'aide d'un générateur;
- envoyer un courant haute fréquence de travail, par temps d'application de quelques fractions de secondes à quelques secondes.

D'autres caractéristiques et avantages de la présente invention apparaîtront mieux à la lecture de la description suivante donnée à titre d'exemple non limitatif des formes possibles de réalisation de l'invention, en regard des dessins ci-joints, et qui fera bien comprendre comment l'invention peut être réalisée.

Sur les dessins :
- la figure 1 est une coupe de principe d'un bistouri à plasma de l'art antérieur;
- la figure 2 est une coupe de principe de bistouri à plasma selon l'invention;
- la figure 3 est une vue partiellement éclatée d'un bistouri à plasma selon un mode de réalisation de l'invention.

La figure 1 a déjà été décrite comme illustrant un dispositif de l'art antérieur. La figure 2 est similaire à la figure 1, mais pour un bistouri à plasma selon l'invention. Sur cette figure, deux électrodes sensiblement linéaires et pointues en leur extrémité 11 et 12, formant de préférence un angle aigu, sont disposées l'une par rapport à l'autre et soumises à des différences de potentiel électrique alternatives de façon que des arcs électriques 13 se produisent entre leurs extrémités. Un courant de fluide, arrivant le long des électrodes 11 et 12, par des conduits respectivement 17 et 18 qui les entourent, et matérialisé par les flèches 14, est envoyé sur les arcs électriques. (et) Il en résulte la production d'un jet de plasma 15, sensiblement le long de l'axe bissecteur des axes longitudinaux des électrodes 11 et 12.

Les conduits 17 et 18 peuvent ainsi servir de porte-électrodes et être au même potentiel que les électrodes. La disposition de la figure 2 est avantageuse, car le fluide passant par les conduits 17 et 18 peut servir à la production du jet de plasma, mais aussi au refroidissement des électrodes. Selon une variante, une gaine peut entourer chaque porte-électrodes et conduire le fluide entre un espace prévu entre ladite gaine et le porte-électrodes, ce qui permet de réduire le jeu entre une électrode et son porte-électrodes.

La disposition des électrodes et la forme sensiblement linéaire -faible section par rapport à la longueur- ou pointue de leurs extrémités favorise le phénomène dit d'effet de pointe, en vertu duquel l'arc électrique ne suit pas une ligne droite entre les extrémités des électrodes, mais a tendance à partir de chaque extrémité d'électrode selon son axe longitudinal.

De plus, le courant de fluide a tendance à souffler l'arc en aval de ce courant, favorisant lui aussi la courbure de l'arc électrique vers le champ opératoire.

A la différence des dispositifs de l'art antérieur, le bistouri à plasma selon l'invention est alimenté en courant alternatif, ce qui a l'avantage de provoquer une usure symétrique des électrodes, au lieu de l'effet de creusement et d'affutage par l'arc électrique d'électrodes alimentées en courant continu. De plus, la chaleur est répartie également entre les électrodes. L'usage préférentiel de haute (ou moyenne) fréquence favorise une excitation à distance des fluides employés, ainsi que le chauffage de la surface sur laquelle sont appliquées les électrodes par effet Joule et/ou par pertes diélectriques suivant les matières traitées, tous ces phénomènes concourant à éviter au maximum l'usure des électrodes avec le refroidissement préférentiellement coaxiale de celles-ci.

Chaque électrode peut être constituée par exemple par un fil d'un métal qui résiste bien aux très hautes températures, comme le tungstène, ou tout autre corps suffisamment conducteur, réfractaire et peu ou pas attaqué à haute température dans le ou les fluides envoyés. L'utilisation d'électrodes filiformes implique préférentiellement l'application de faibles intensités électriques sous tension élevée.

Selon l'invention, le dispositif est alimenté par un courant électrique réglable et/ou régulé. En effet, il est par exemple nécessaire d'adapter la tension aux bornes des électrodes en fonction de la longueur de l'arc électrique, et celui-ci étant comme on l'a vu en quelque sorte déformé vers l'extérieur par le passage du fluide, cette longueur peut varier. De plus, l'amorçage de l'arc électrique peut être obtenu de façon conventionnelle par rapprochement des électrodes, mais aussi par une augmentation de la tension aux bornes des électrodes, ou encore par l'application d'un courant modulé en amplitude. D'autre part, les différentes natures de matériaux traités selon leur conduction de surface ou leurs propriétés diélectriques font varier les réglages ; la régulation apporte une plus grande facilité d'emploi.

Le fluide peut être un gaz, notamment de l'air ou un gaz rare, mais aussi tout autre fluide adapté. De plus, on peut être amené à utiliser plusieurs fluides différents.

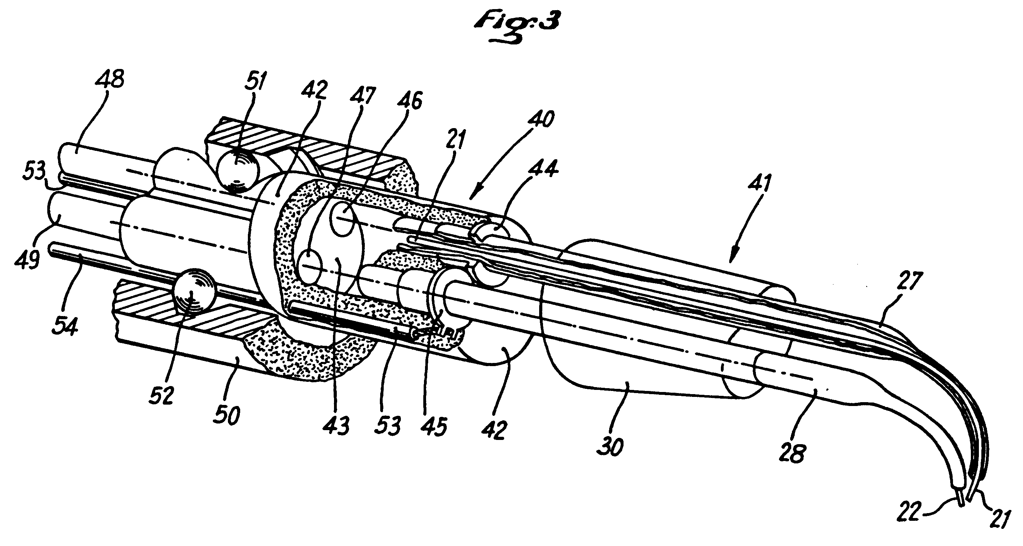

Sur la figure 3, un bistouri à plasma selon l'invention comprend un porte-outil 40 et un ensemble porte-électrodes 41. L'ensemble porte-électrodes 41 se compose princialement de deux conduits cylindriques porte-électrodes 27 et 28 se terminant par une partie avant arrondie ou coudée de diamètre moindre. Dans chaque conduit 27, 28, on a placé une électrode, respectivement 21, 22, constituée d'un fil de tungstène dont le diamètre est légèrement inférieur à celui de ladite partie arrondie. Avantageusement, les deux conduits porte-électrodes 27 et 28 sont pris en leur partie centrale par un manchon isolant 30.

Le porte-outil 40 comprend un boîtier étanche 42 à l'intérieur duquel se trouve une chambre 43 et qui comporte dans l'une de ses parois, dite avant, deux ouvertures munies chacune d'une douille 44, 45, définissant dans le boîtier étanche 42 deux passages cylindriques entre ladite chambre 43 et l'extérieur, dont le diamètre correspond sensiblement au diamètre extérieur des deux conduits porte-électrodes 27 et 28. Dans la paroi opposée à ladite paroi avant, que l'on appellera paroi arrière du boîtier étanche 42, sont prévues deux ouvertures 46 et 47, dans lesquelles débouchent deux tubes 48, 49, soit un tube dans chaque ouverture. Des moyens d'obturation, qui dans l'exemple de la figure 3 sont du type à billes 51, 52 et bague de commande translatable 50, sont prévus immédiatement en amont des ouvertures 46, 47, pour permettre ou non à des fluides sous pression dans les tubes 48, 49 de passer dans la chambre 43.

Les moyens d'obturation pourraient, dans un mode de réalisation avantageux de l'invention, comprendre un manchon rigide traversé par au moins deux tubes d'arrivée de fluide rendus très déformables à l'intérieur dudit manchon, de façon qu'une pression élevée dans l'un des tubes obture les autres en les écrasant contre les parois intérieures dudit manchon rigide. Dans ce mode de réalisation, il n'y a aucune commande manuelle au niveau de l'outil, les arrivées de fluide ou mises sous pression des tubes pouvant être commandées au moyen de pédales.

Les conduits porte-électrodes 27 et 28 sont introduits dans lesdites douilles 44 et 45 respectivement. Lorsqu'un fluide sous pression se trouve dans la chambre 43, il s'écoule dans les conduits porte-électrodes 27 et 28, autour des électrodes 21 et 22.

Lesdites douilles conductrices 44, 45 sont connectées, par exemple par soudage ou sertissage, à des fils conducteurs isolés respectivement 53, 54 qui, dans l'exemple de la figure 3, sont noyés dans l'épaisseur des parois du boîtier étanche 42, ressortent au niveau de sa paroi arrière et sont raccordés à un générateur de courant par l'intermédiaire d'un transformateur élévateur de tension, tous deux non représentés sur la figure. Les douilles 44 et 45 et lesdits conduits porte-électrodes 27, 28 sont fabriquées à partir d'un matériau conducteur de l'électricité. Comme, par construction, chaque douille 44, 45 est en contact avec le conduit porte-électrodes introduit dedans, respectivement 27, 28, et que chaque électrode est en contact avec son conduit porte-électrodes, respectivement 21, 22, une différence de potentiel entre les conducteurs isolés 53, 54 est transmise entre les électrodes 21, 22. Par ailleurs, le tube 48 est mis sous pression d'air. L'autre conduit 49 n'est pas forcément utilisé, et n'est d'ailleurs pas nécessaire au fonctionnement de l'invention. Sa présence constitue une variante avantageuse permettant de faire passer le cas échéant un autre fluide que de l'air dans les conduits porte-électrodes.

Selon une variante, on peut supprimer lesdits moyens d'obturation, et un fluide, ici de l'air, s'écoule en permanence le long des électrodes.

Le dispositif de l'invention comporte de préférence un moyen, notamment de jet d'eau, non représenté, lié au dispositif, permettant d'amener de l'eau au voisinage des extrémités des électrodes. L'eau peut être apportée par au moins un des conduits porte-électrodes.

Les porte-électrodes peuvent être également de section elliptique polygonale, etc., toute forme permettant la fonction étant satisfaisante, éventuellement le fil peut être travaillé pour favoriser son frottement ou sa rigidité, ondulé, muni d'une nervure, etc. Le diamètre des électrodes filiformes étant inférieur au diamètre des porte-électrodes, la rétention de ces électrodes peut se faire par frottement grâce à des irrégularités géométriques internes des porte-électrodes, par exemple un coude. Cette rétention peut se faire également par des irrégularités externes de ces électrodes fililformes, par exemple une ondulation.

Cet ensemble porte-électrodes formant tête peut être éventuellement amovible, interchangeable ou à usage unique. Plus généralement, lesdits conduits porte-électrodes et/ou lesdites électrodes peuvent être consommables, interchangeables et remplaçables. Les porte-électrodes 27 et 28 peuvent être rectilignes convergents pour des applications particulières, mais, en usage dentaire, la forme dite "contre-angle" ou angle droit ou similaire sera avantageusement adoptée pour une meilleure facilité d'emploi. La forme de l'instrument tel que représenté sur la figure 3 permet l'observation par dessus pendant le travail, ce qui n'est pas possible avec les instruments dentaires rotatifs. D'une façon générale, et de préférence, les deux électrodes forment des courbes planes définissant chacunes un plan, ces plans formant un angle entre eux de façon telle que l'observation du champ opératoire soit rendue possible depuis l'intérieur dudit angle de préférence aigu, afin que cette observation puisse se faire selon un axe de vision sensiblement perpendiculaire à la surface de l'objet sur lequel on travaille.

L'ensemble porte-électrodes peut être facilement interchangeable, par exemple par simple friction, pour des raisons de remplacement d'électrodes, de changement de configuration de cette partie terminale ainsi que d'aseptie ; cette dernière raison est justifiée pour des applications médicales.

Selon la présente invention, un procédé de découpage et de traitement de matières dures ou molles, notamment des tissus vivants, avec le dispositif de la figure 3 comprend les étapes suivantes:
- envoyer éventuellement une petite quantité d'eau à la sortie des porte-électrodes, afin d'améliorer la conductibilité de l'espace entre les électrodes, et faciliter ainsi l'amorçage d'un arc électrique entre elles; l'eau peut également, si nécessaire, servir à refroidir le tissu à traiter;
- ouvrir l'alimentation en air pour mettre la chambre 43 sous pression d'air, et provoquer ainsi un courant d'air le long et autour des électrodes;
- établir un courant haute fréquence pour amorcer l'arc électrique, éventuellement modulé en amplitude ou en fréquence, à l'aide du générateur;
- adapter le cas échéant la modulation du courant au travail à effectuer;
- envoyer un courant haute fréquence de travail, par temps d'application de quelques fractions de secondes à quelques secondes.

En ce qui concerne la dernière étape ci-dessus, le travail se fait par périodes de quelques fractions de secondes à quelques secondes, entrecoupées de temps de relaxation qui peuvent être mis à profit pour refroidir la matière par eau, spray ou autre moyen.

Des moyens peuvent être prévus pour le réglage des différences de potentiel électrique, et des temps de relaxation entre périodes d'application desdites différences de potentiel, ainsi que des durées desdites périodes et des moments d'action du ou des différents fluides.

Le générateur de courant est avantageusement pourvu de moyens de réglages et/ou de programmation permettant notamment de faire varier l'intensité du courant et/ou sa tension et/ou sa modulation. On a vu que le courant de fluide passant à travers l'arc électrique avait tendance à souffler l'arc en aval dudit courant. Ceci a pour effet de l'allonger, comme si on écartait les électrodes. Pour compenser cet allongement, il est nécessaire d'augmenter la tension aux bornes des électrodes. On peut même prévoir, sans sortir du cadre de la présente invention, d'adjoindre au dispositif un moyen d'asservissement capable de faire varier automatiquement la tension et/ou le courant en provenance du générateur en fonction du débit d'air.

Avantageusement, le dispositif est muni immédiatement en arrière du porte-outil 40, d'un petit transformateur élévateur de tension. En effet, compte-tenu des fréquences utilisées, soit de quelques dizaines de kilohertz à quelques mégahertz, un tel transformateur est peu encombrant et, selon l'invention, il est important de limiter le trajet du courant haute tension. Conformément à une caractéristique importante de l'invention, le transformateur est incorporé à une poignée du bistouri. Les tensions utilisées vont de quelques centaines de Volts à quelques milliers de Volts, suivant les difficultés d'amorçage de l'arc, lesquelles difficultés étant elles-mêmes fonction des électrodes (configuration, éloignement), du ou des fluides envoyés et de la surface et de la matière traitée. A titre d'exemple, on peut prendre un rapport élévateur de quatre pour le transformateur, en partant d'une tension d'alimentation haute fréquence de 220 volts. L'intensité du courant électrique est limitée par la durée de vie des électrodes ou la rapidité de leur consommation.

Le transformateur incorporé à la partie mobile du dispositif est pratiquement indispensable. En effet, si il était placé à distance du bistouri, il y aurait affaiblissement dans le câble et perte de puissance et trop peu d'énergie parviendrait aux électrodes. D'autre part, sauf à utiliser une isolation complémentaire encombrante, il y aurait danger de brûlure à proximité du fil. Pour réduire l'encombrement du transformateur, on peut le réaliser à partir d'un noyau en bâtonnet cylindrique entouré par des enroulements, si l'on est prêt à accepter la présence d'un champ magnétique important autour. Cet inconvénient n'existe pas avec un circuit magnétique fermé -enroulements sur tore(s) et fermeture du circuit- mais alors le transformateur est plus gros. En tout état de cause, les fréquences en jeu sont telles que les dimensions du transformateur restent faibles.

Le ou les fluides peuvent passer par l'intérieur du transformateur pour le refroidir.

En applications dentaires ou médicales au moins, la haute ou moyenne fréquence est nécessaire pour éviter tout risque d'électrocution. Une alimentation par impulsions est possible.

Pour obtenir l'amorçage de l'arc électrique, si nécessaire, outre le conventionnel rapprochement des électrodes, on peut agir par adjonction d'un fluide suffisamment conducteur au départ, tel qu'un brouillard d'amorçage, Un amorçage par un pic de tension délivré, par exemple, par une céramique plézoélectrique est également envisageable, mais avec des réserves pour un usage médicodentaire.

On peut également adjoindre au dispositif un moyen d'éclairage, constitué par exemple d'une ampoule électrique muni d'un conduit de lumière afin de faciliter un guidage manuel pré et post opératoire en raison de la nécessaire protection oculaire.

Selon l'invention, le dispositif peut comprendre un coffret de commande permettant de centraliser tous les réglages nécessaires au bon fonctionnement du dispositif. Un tel coffret permettrait notamment de régler :
- le débit de gaz;
- le débit d'eau;
- la tension électrique ;
- l'intensité de la lumière, celle-ci pouvant être du type lumière froide et/ou être véhiculée par un conduit de lumière du type fibre optique, par exemple ;
- la modulation du courant électrique (taux, fréquence);
- le cas échéant les réglages relatifs aux impulsions (durées, temps de relaxation, etc...). On entend ici par impulsions les courtes périodes pendant lesquelles sont produits des arcs électriques.

Selon un mode de réalisation avantageux de l'invention, un système de commande associé à une chambre interne au manche permet d'envoyer alternativement par la lumière d'au moins un des porte-électrodes soit le fluide à envoyer si nécessaire dans les temps de fonctionnement, soit un fluide absorbant particulièrement la chaleur, par exemple de l'eau, pendant les temps de repos. Ledit système de commande réalise une programmation réglable des courants électriques et des fluides pour satisfaire les séquences opératoires.

On peut avoir une ou plusieurs électrodes enrobées pour un apport de matériau, ou bien un fil de matériau placé au voisinage d'électrodes pour ce même usage.

On peut être amené à prévoir une excitation annexe du plasma, notamment électrique ou chimique. Pour l'excitation électrique, un courant continu peut être superposé à un courant haute fréquence, même en usage dentaire ou médical sous réserve que le courant soit inférieur à une valeur nuisible ; quant à l'excitation chimique, des fluides différents peuvent servir au refroidissement avant qu'une réaction exothermique ne se produise vers le point d'application.

Lors d'un fonctionnement discontinu ou impulsif, les temps "morts" peuvent être employés à refroidir l'objet avant et/ou après l'action du plasma. Ce refroidissement peut se faire notamment par les mêmes conduits ou buses que ceux décrits, les traces de liquide et/ou de gaz (chargés ou non) pouvant servir lors de la remise en route à favoriser l'amorçage de l'arc initiateur du plasma.

On distingue plusieurs variantes possibles du dispositif de l'invention, tout d'abord dans le cas d'électrodes symétriques :
- les dispositifs à électrodes convergentes pleines à usage dentaire, médical et industriel : l'arc apparaissant entre les parties les plus proches des électrodes est soufflé par un fluide, par exemple de l'air, qui le projette. Les électrodes sont éventuellement rapprochées pour obtenir l'amorçage ;
- les dispositifs à électrodes convergentes creuses à usage dentaire, médical et industriel : un refroidissement interne des électrodes avec un fluide circulant à l'intérieur servant à la production du plasma peut se substituer ou s'ajouter à celui d'une ou plusieurs buses projetant un fluide éventuellement chargé tel qu'un aérosol (phase dispersée solide ou liquide). Les électrodes sont éventuellement rapprochées pour obtenir l'amorçage ;
- les dispositifs à porte-électrodes soufflants creux et électrodes consommables à usage dentaire, médical ou industriel : c'est l'exemple de la figure 3, où des porte-électrodes convergents contiennent les électrodes en fil, consommables, à action physique et/ou chimique. Le diamètre de ces électrodes est un peu inférieur à la lumière des porte-électrodes, permettant ainsi le passage du ou des fluides réfrigérants participant à la production de plasma, et éventuellement réactifs avec l'objet traité ou entre eux. Les électrodes sont éventuellement rapprochées pour obtenir l'amorçage. Le fil consommable se prolonge, si nécessaire en amont des porte-électrodes, pour assurer une réserve à l'usage. Un système mécanique d'avance automatique ou manuel des électrodes peut être adjoint. Le ou les fluides peuvent être amenés par un ou des conduits concentriques aux porte-électrodes, une ou plusieurs buses peuvent s'ajouter ou se substituer en projetant un ou des fluides éventuellement chargés (phase dispersée, solide ou liquide) ;
- les dispositifs à électrodes multiples à usage dentaire, médical et industriel : appareils à n électrodes (n entier naturel) avec une alimentation en courant monophasé ou polyphasé amenant à la réalisation d'un plasma alternatif ou tournant qui peut être soufflé et/ou guide par des champs magnétiques ou électriques continus, alternatifs ou pulsés.

Dans le cas de dispositifs à électrodes dissymétriques, voici quelques configurations possibles, qui restent dans le cadre de la présente invention :
- configuration plane à usage dentaire, médical et industriel : une première électrode est placée en avant par rapport à une deuxième, qui est légèrement en retrait et coudée ou inclinée vers la première, avec une éventuelle dissymétrie d'alimentation et/ou de débit de fluides. Cette configuration permet de privilégier l'activité d'une des électrodes. On peut avoir plusieurs électrodes semblables à la deuxième, disposées autour de la première électrode. Les électrodes sont éventuellement rapprochées pour obtenir l'amorçage. Les conduits porte-électrodes soufflants pouvant être remplacés par une ou plusieurs buses.
- configuration volumique à usage dentaire, médical et industriel : une première électrode linéaire entourée en son extrémité par une deuxième électrode en forme d'anneau, avec une éventuelle dissymétrie d'alimentation et/ou de débit de fluides amène à circonscrire l'action grâce à l'anneau formé par la deuxième électrode, laquelle peut se présenter également sous la forme d'une plaque métallique percée d'un trou. Les électrodes sont éventuellement rapprochées pour obtenir l'amorçage. Ici aussi, les porte-électrodes soufflants peuvent être remplacés par une ou plusieurs buses.

Les applications possibles d'un tel dispositif sont nombreuses :
- dans le domaine de la chirurgie dentaire, on peut traiter l'émail et la dentine de façon comparable à celle d'un laser. On peut également utiliser l'émission de lumière qui accompagne la production de plasma pour activer les photopolymérisations de substances, notamment certaines résines, utilisées pour le remplissage de trous pratiqués dans des dents dans le cadre de soins dentaires ou pour réaliser des prothèses dentaires car les températures de couleur de la lumière émise sont adaptées à un tel usage, sous réserve de prévoir des filtres, lesquels filtres peuvent être refroidis par le ou les fluide émis;
- dans le domaine de la médecine, l'exérèse de tissus ; la miniaturisation de l'appareil permettrait une utilisation de type endoscopique pour intervenir à l'intérieur d'un organisme vivant; l'utilisation de l'effet capacitif du système peut alors être utilisé pour réaliser un circuit oscillant accordé de façon à réduire les pertes résultant de la nécessité d'éloigner le transformateur.
- dans les autres domaines, on peut prévoir de nombreux développements possibles, notamment pour des opérations de gravure, de découpage, de soudure, ou de recharge en matériaux, traitement des matières composites, etc...

De plus, si on revient à l'exemple de la figure 3, on peut considérer que le porte-outil 40 peut alimenter d'autres outils que l'ensemble porte-électrodes 41. On a vu que l'on pouvait utiliser l'émission de lumière du plasma pour activer des réactions de photopolymérisation. On peut également utiliser le dispositif de la figure 3 pour pratiquer des électrosections de tissus traditionnelles, monopolaires ou bipolaires, en utilisant une seule ou deux électrodes. L'effet capacitif du transformateur permettrait, moyennant certaines précautions, l'usage en bistouri électrique bipolaire monoactif éventuellement avec une alimentation des deux fils haute fréquence du cordon sur la même borne active, l'autre borne servant éventuellement à une électrode indifférente placée près du patient. La configuration en bistouri ou microchalumeau à plasma serait en fait, selon une extension des termes utilisés en électrochirurgie, bipolaire biactive mésioactive à très haute température.

Le dispositif est donc polyvalent, puisqu'il réunit au moins trois instruments en un seul (bistouri à plasma, bistouri électrique, source de lumière pour photopolymérisation).

## Revendications

1. Bistouri à plasma (40, 41) comportant une poignée, un câble d'alimentation électrique et au moins deux électrodes espacées (11, 12, 21, 22) soumises à des différences de potentiel électrique telles que des arcs électriques (13) se produisent entre elles et un moyen pour produire au moins un courant de fluide (14) traversant une région de l'espace où lesdits arcs électriques (13) ont lieu, lesdites électrodes étant adaptées à être mises sensiblement au contact d'une zone à traiter, lesdites différences de potentiel étant alternatives, de moyenne ou haute fréquence, caractérisé en ce que les extrémités desdites électrodes sont filiformes ou pointues et forment entre elles un angle aigu, et un transformateur élévateur de tension est incorporé au bistouri pour délivrer lesdites différences de potentiel alternatives, lesdits courants de fluide (14) arrivant depuis l'intérieur dudit angle, ou le long d'au moins une desdites électrodes.

2. Bistouri selon la revendication 1, dans lequel chacune desdites électrodes est constituée d'un fil de matériau conducteur de l'électricité (21, 22), et est placée dans un conduit porte-électrodes (17, 18, 27, 28) dont le diamètre intérieur est légèrement supérieur au diamètre de l'électrode, l'extrémité de l'électrode dépassant du conduit, et en ce que lesdits fluides sont véhiculés par lesdits conduits porte-électrodes.

3. Bistouri selon la revendication 2, dans lequel lesdits conduits porte-électrodes et/ou lesdites électrodes sont consommables, interchangeables et remplaçables.

4. Bistouri selon la revendication 2, dans lequel les conduits porte-électrodes (27, 28) communiquent avec une chambre (43) pouvant être mise sous pression d'au moins un desdits fluides à l'aide de moyens d'obturation (50, 51, 52) prévus entre ladite chambre et au moins un tube (48, 49) d'arrivée de fluide.

5. Bistouri selon l'une des revendications précédentes, dans lequel des moyens sont prévus pour le réglage ou la programmation des différences de potentiel électrique, et des temps de relaxation entre périodes d'application desdites différences de potentiel, ainsi que des durées desdites périodes.

6. Bistouri selon l'une des revendications précédentes, dans lequel des moyens sont prévus pour régler les débits de fluide.

7. Bistouri selon l'une des revendications précédentes, dans lequel les deux électrodes forment des courbes planes définissant chacunes un plan, ces plans formant un angle entre eux de façon telle que l'observation du champ opératoire soit rendue possible depuis l'intérieur dudit angle, afin que cette observation puisse se faire selon un axe de vision sensiblement perpendiculaire à la surface de l'objet sur lequel on travaille.

8. Bistouri selon la revendication 1, dans lequel les électrodes sont creuses et conduisent lesdits fluides.

9. Bistouri selon l'une des revendications précédentes, dans lequel des moyens sont prévus pour amener de l'eau au voisinage des extrémités des électrodes.

10. Bistouri selon la revendication 9 rattachée à la revendication 2, dans lequel l'eau est apportée par au moins un des conduits porte-électrodes.

11. Bistouri selon l'une des revendications 4 à 10, dans lequel lesdits moyens d'obturation comprennent un manchon rigide traversé par au moins deux tubes d'arrivée de fluide rendus très déformables à l'intérieur dudit manchon, de façon qu'une pression élevée dans l'un des tubes obture les autres en les écrasant contre les parois intérieures dudit manchon rigide.

12. Procédé de découpe ou de traitement de matières dures ou molles à l'aide d'un bistouri selon la revendication 4, caractérisé en ce qu'il comprend les étapes suivantes :
- ouvrir l'alimentation en air pour mettre la chambre (43) sous pression d'air, et provoquer ainsi un courant d'air le long ou autour des électrodes;
- établir un courant haute fréquence pour amorcer l'arc électrique à l'aide d'un générateur;
- envoyer un courant haute fréquence de travail, par temps d'application de quelques fractions de secondes à quelques secondes.

13. Procédé selon la revendication 12, comprenant l'étape préliminaire d'envoyer une petite quantité d'eau à la sortie des porte-électrodes, afin d'améliorer la conductibilité de l'espace entre les électrodes, et faciliter ainsi l'amorçage d'un arc électrique entre elles.

14. Procédé selon la revendication 12 ou la revendication 13, dans lequel ledit courant haute fréquence est modulé en amplitude ou en fréquence.

## Claims

1. A plasma scalpel (40, 41) comprising a grip, an electrical feeding cable and at least two spaced-apart electrodes (11, 12, 21, 22) subjected to differences in electrical potential such that electric arcs (13) occur therebetween, and means for producing at least one fluid flow (14) passing through a region of space where said electric arcs (13) occur, said electrodes being arranged to substantially contact an area to be treated, said potential differences are alternating differences at medium or high frequency, wherein the ends of said electrodes are substantially filiform or pointed and form an acute angle therebetween, and wherein a voltage step-up transformer is incorporated to deliver said alternating potential differences, said fluid flows (14) arriving from inside said angle or along at least one of said electrodes.

2. A scalpel according to claim 1, wherein each of said electrodes is constituted by a wire of electrically conductive material (21, 22), and is placed in an electrode-carrying duct (1è, 18, 27, 28) whose inside diameter is slightly greater than the diameter of the electrode, with the end of the electrode projecting beyond the end of the duct, and wherein said fluids are conveyed by said electrode-carrying ducts.

3. A scalpel according to claim 2, wherein said electrode-carrying ducts and/or said electrodes are consumable, interchangeable, and replaceable.

4. A scalpel according to claim 2, wherein the electrode-carrying ducts (27, 28) communicate with a chamber (43) suitable for being put under pressure by at least one of said fluids under the control of closure means (50, 51, 52) provided between said chamber and at least one fluid admission tube (48, 49).

5. A scalpel according to one of the preceeding claims, wherein means are provided for adjusting or programming the electrical potential differences and the relaxation times between the periods during which said potential differences are applied, said means also serving to adjust the durations of said periods.

6. A scalpel according to one of the preceeding claims, wherein means are provided for adjusting the fluid flow rates.

7. A scalpel according to one of the preceeding claims, wherein the two electrodes form plane curves each defining a plane, said planes forming an angle therebetween suitable for enabling the operating field to be observed from inside said angle, thereby enabling said operation to take place along an observation axis which is substantially perpendicular to the surface of the object on which work is being performed.

8. A scalpel according to claim 1, wherein the electrodes are hollow and convey said fluids.

9. A scalpel according to one of the preceeding claims, wherein means are provided for conveying water to the vicinity of the ends of the electrodes.

10. A scalpel according to claim 9 depending from claim 2, wherein the water is conveyed via at least one of the electrode-carrier ducts.

11. A scalpel according to one of the claims 4 to 10, wherein said closure means comprise a rigid sleeve a rigid sleeve having at least two fluid admission tubes which are highly deformable inside the sleeve so that a high pressure in one of the tubes closes the other tube(s) by compression against the inside walls of said rigid sleeve.

12. A method of cutting or treating hard or soft substances by means of a scalpel according to claim 4, the method comprising the following steps:
switching on the air feed to put chamber (43) under air pressure, thereby causing air to flow along and around the electrode;
establishing high frequency current to strike the electric arc, said current being provided by the generator;
applying a high frequency working current for application periods lasting a fraction of a second to a few seconds.

13. A method according to claim 12, comprising the preliminary step of applying a small quantity of water to the output from the electrode-carriers in order to improve the conductivity of the space between the electrodes and thus facilitate striking an electric arc therebetween.

14. A method according to claim 12 or claim 13, wherein said high frequency current is frequency or amplitude modulated.

## Patentansprüche

1. Plasma-Operationsmesser (40, 41) bestehend aus einem Griff, einem Stromzufuhrkabel und mindestens zwei auseinanderliegenden Elektroden (11, 12, 21, 22), die elektrischen Spannungsunterschieden ausgesetzt sind, so daß Lichtbögen (13) zwischen ihnen entstehen und die Möglichkeit gegeben ist, mindestens einen Flüssigkeitsstrom (14) zu erzeugen, der einen Teil des Raumes durchläuft, in dem die genannten Lichtbögen (13) entstehen, wobei die genannten Elektroden so eingerichtet wurden, daß sie genau mit einem zu behandelnden Bereich in Kontakt gebracht werden können; bei den genannten Spannungsunterschieden handelt es sich um Wechselstrom, Hochfrequenzstrom oder Strom mittlerer Frequenz.
dadurch gekennzeichnet, daß
die Enden der genannten Elektroden fadenförmig oder spitz sind und einen spitzen Winkel bilden, und ein spannungserhöhender Transformator für die Wechselstromspannungsunterschiede in das Operationsmesser eingebaut ist, wobei die genannten Flüssigkeitsströme (14) aus dem Inneren des genannten Winkels kommen oder entlang mindestens einer der genannten Elektroden.

2. Operationsmesser gemäß Anspruch 1, bei dem jede der genannten Elektroden aus einem Draht aus stromleitendem Material (21, 22) ist und in eine Elektrodenträgerleitung (17, 18, 27, 28) eingebracht ist, deren Innendurchmesser geringfügig über dem Elektrodendurchmesser liegt, wobei das Ende der Elektrode über die Leitung hinausgeht und die genannten Flüssigkeiten durch die genannten Elektrodenträgerleitungen transportiert werden.

3. Operationsmesser gemäß Anspruch 2, bei dem die genannten Elektrodenträgerleitungen und/oder die genannten Elektroden aufgebraucht, ausgetauscht und ersetzt werden können.

4. Operationsmesser gemäß Anspruch 2, bei dem die Elektrodenträgerleitungen (27, 28) mit einer Kammer (43) verbunden sind, die mit mindestens einer einer genannten Flüssigkeiten unter Druck gesetzt werden kann, und zwar mit Hilfe von Verschlußmöglichkeiten (50, 51, 52) zwischen genannter Kammer und mindestens einer Flüssigkeitszufuhrleitung (48, 49).

5. Operationsmesser gemäß einem der vorgenannten Ansprüche, das mit einer Regulierung oder Programmierung für die elektrischen Spannungsunterschiede ausgestattet ist, sowie für Ruhezeiten zwischen den Einsatzzeiten der genannten Spannungsunterschiede und für die Dauer der genannten Einsatzzeiten.

6. Operationsmesser gemäß einem der vorgenannten Ansprüche, mit Ausstattung zur Regulierung der Flüssigkeitsabgabe.

7. Operationsmesser gemäß einem der vorgenannten Ansprüche, bei dem die beiden Elektroden flächenhafte plane Krümmungen bilden, wobei die Flächen einen Winkel bilden, der es erlaubt aus dem Inneren des Winkels das Operationsfeld einzusehen, so daß die Sehachse für eine Beobachtung genau senkrecht auf die Oberfläche des Objekts, auf dem man arbeitet, trifft.

8. Operationsmesser gemäß Anspruch 1, bei dem die Elektroden hohl sind und die genannten Flüssigkeiten leiten.

9. Operationsmesser gemäß einem der vorgenannten Ansprüche, das derart ausgestattet ist, daß Wasser in die Nähe der Enden der Elektroden herangeführt werden kann.

10. Operationsmesser gemäß Anspruch 9 zusätzlich zu Anspruch 2, bei dem das Wasser durch mindestens eine der Elektrodenträgerleitungen herangeführt wird.

11. Operationsmesser gemäß einem der Ansprüche 4 bis 10, bei dem zu den genannten Verschlußmöglichkeiten eine steife Manschette gehört, durch die mindestens zwei Flüssigkeitszufuhrleitungen führen, die, äußerst elastisch gemacht, im Inneren der Manschette sind, derart, daß ein erhöhter Druck in einer der Leitungen die anderen verschließt, indem sie sie gegen die Innenwände der genannten steifen Manschette plattdrückt.

12. Verfahren zum Schneiden oder Bearbeiten von hartem oder weichem Material mit Hilfe eines Operationsmessers gemäß Anspruch 4,
dadurch gekennzeichnet, daß
das Verfahren folgende Schritte umfaßt:
- Öffnen der Luftzufuhr, um die Kammer (43) unter Luftdruck zu setzen und damit einen Luftzug entlang oder um die Elektroden zu erzeugen;
- Hochfrequenzstrom anlegen, um den Lichtbogen mit Hilfe eines Generators zu zünden;
- mit Hochfrequenzarbeitsstrom beschicken, pro Einsatzzeit für einige Teilsekunden bis zu einigen Sekunden.

13. Verfahren gemäß Anspruch 12; hierzu gehört der einleitende Schritt, bei dem eine kleine Menge Wasser zum Ausgang der Elektrodenträger geschickt wird, um die Leitfähigkeit des Raumes zwischen den Elektroden zu verbessern und damit die Lichtbogenzündung für einen Elektrodenüberschlag zu erleichtern.

14. Verfahren gemäß Anspruch 12 oder Anspruch 13, bei dem Amplitude und Frequenz des genannten Hochfrequenzstroms reguliert werden.
